# EUROPEAN PATENT SPECIFICATION

(11) **EP 2 205 991 B1**
(45) Date of publication and mention of the grant of the patent: **29.08.2018**
(21) Application number: 08845807.0
(22) Date of filing: 29.10.2008
(51) Int. Cl.: G01S 15/89, B06B 1/06, G10K 11/00, A61B 8/00

(54) **ULTRASOUND ASSEMBLY INCLUDING MULTIPLE IMAGING TRANSDUCER ARRAYS**
ULTRASCHALL-EINHEIT MIT MEHREREN WANDLERARRAYS ZUR BILDERZEUGUNG
ENSEMBLE ULTRASONORE COMPRENANT DE MULTIPLES RÉSEAUX DE TRANSDUCTEURS D'IMAGERIE

(30) Priority: 29.10.2007 US 983258 P
(43) Date of publication of application: 14.07.2010
(73) Proprietor: Koninklijke Philips N.V., 5656 AE Eindhoven (NL)
(72) Inventor: ROBINSON, Brent S., Briarcliff Manor, NY 10510-8001 (US)
(74) Representative: van Velzen, Maaike Mathilde
(86) International application number: PCT/IB2008/054503
(87) International publication number: WO 2009/057062

(56) References cited:
- WO-A-00/30540
- US-A- 4 603 702
- US-A1- 2007 016 058
- US-B1- 6 669 638

## Description

### Technical Field

The present disclosure relates to transducer-based systems for ultrasonic diagnostic imaging. More particularly, the present disclosure is directed to ultrasonic transducer apparatus/systems and related methods that include and/or facilitate use of a plurality of discrete ultrasonic image data acquisition transducer arrays that are disposed with respect to a single ultrasonic transducer assembly.

### Background

Ultrasonic diagnostic imaging systems allow medical professionals to examine internal structures of patients without invasive exploratory surgery. Ultrasonic diagnostic imaging systems typically include a variety of types of transducer assemblies each having different image data acquisition capabilities, operating characteristics, and/or modes of operation. The transducer assemblies could be connected through cables to a host system.

Ultrasonic examinations often require use of more than one type of transducer assembly. For example, a sonographer could use a first ultrasonic transducer assembly having a curved array of transducer elements to perform a first portion of an ultrasonic examination. The sonographer could then remove the first ultrasonic transducer assembly and replace it with a second ultrasonic transducer assembly having a linear phased array of transducer elements to perform a second portion of the ultrasonic examination. The change of ultrasonic transducer assemblies may be required because the transducer assemblies have different capabilities, such as inter-costal access, for example.

Alternatively, the sonographer could employ a first ultrasonic transducer assembly including an ultrasonic image data acquisition transducer array that operates at a high center frequency, which results in high resolution image data being acquired. The sonographer could then remove the first ultrasonic transducer assembly and replace it with a second ultrasonic transducer assembly that operates at a lower center frequency. The lower frequency ultrasonic image data acquisition transducer array results in lower resolution image data being acquired, however, the lower frequency transducer assembly is capable of achieving greater penetration distances.

In addition, certain ultrasonic diagnostic protocols require that the sonographer change ultrasonic transducer assemblies during an examination. However, changing ultrasonic transducer assemblies may be inconvenient, particularly with mobile ultrasonic diagnostic imaging systems, which require the sonographer to physically transport a variety of ultrasonic transducer assembly to an examination location. Further, transducer assemblies must be placed in an appropriate location after use, which is particularly inconvenient during intra-operative procedures in a sterile field.

Document US 2007/0016058 A1 shows a system and method for controlling an ultrasound probe. The ultrasound probe includes a first transducer array and a second transducer array. The ultrasound probe further includes a transducer array selector component within a housing. The transducer array selector component is configured to generate a control signal based on a user input to selectively activate one of the first transducer array and the second transducer array.

### Summary

The present disclosure relates to an ultrasonic transducer assembly. According to a first aspect, an ultrasonic transducer assembly according to claim 1 is presented. According to a second aspect, an ultrasonic transducer assembly according to claim 5 is presented. The communications assembly could include a cable or a wireless interface.

In some embodiments, the plurality of ultrasonic image data acquisition transducer arrays are comprised of passive arrays of transducer elements. The controller assembly receives transmit waveforms from the host through the communications assembly and provides the transmit waveforms to the selected ultrasonic image data acquisition transducer array. The selected ultrasonic image data acquisition transducer array transmits corresponding acoustic signals and receives echo signals that are reflected by a target. The received echo signals cause the transducer elements to produce corresponding transducer signals that are provided to the controller assembly for transmission by the communications assembly to the host system.

In some embodiments, the plurality of ultrasonic image data acquisition transducer arrays are comprised of multiplexed arrays of transducer elements. The controller assembly receives transmit waveforms and multiplexer control data from the host system through the communications assembly. The controller assembly provides corresponding transmit waveform signals to the multiplexed array indicated by the control data, which transmit corresponding acoustic signals and receives echo signals that are reflected by a target. The received echo signals cause the transducer elements to produce corresponding transducer signals that are provided to the controller assembly for transmission by the communications assembly to the host system.

In some embodiments, the transducer assembly includes a microbeamformer assembly including a plurality of microbeamformers that are connected to one of the plurality of ultrasonic image data acquisition transducer arrays. Each microbeamformer is in electrical communication with the controller assembly. The controller assembly receives transmit waveforms and microbeamformer control data through the communications assembly and provides corresponding transmit waveform signals and control signals to the appropriate microbeamformer, which provides beamformed transmit signals to the selected ultrasonic image data acquisition transducer array. The selected ultrasonic image data acquisition transducer array transmits corresponding acoustic signals and receives echo signals that are reflected by a target. The received echo signals cause the transducer elements to produce corresponding transducer signals that are provided to the microbeamformer assembly for processing. The microbeamformer assembly provides beamformed transducer signals to the controller assembly for transmission by the communications assembly to the host system.

In some embodiments, the controller assembly performs processing operations on signals provided by the transducer elements prior to transmission to the host system. Examples of such processing operations include secondary beamforming operations, signal conditioning, band-pass filtering, detection, post-filtering operations, analog to digital operations, and/or compression operations.

### Brief Description of the Drawings

To assist those of skill in the art in making and using the disclosed transducer assemblies and related methods, reference is made to the accompanying figures, wherein:
FIG. 1 is a schematic depiction of an exemplary ultrasonic imaging system made in accordance with the present disclosure;
FIG. 2 is a schematic depiction of the exemplary ultrasonic transducer assembly of FIG. 1;
FIG. 3 is a schematic depiction of another exemplary ultrasonic imaging system made in accordance with the present disclosure; and
FIG. 4. is a schematic depiction of the exemplary ultrasonic transducer assembly of FIG. 3.

### Description of Exemplary Embodiment(s)

In accordance with the exemplary embodiments of the present disclosure, an ultrasonic transducer assembly is provided for anatomical imaging that includes a housing, a plurality of ultrasonic image data acquisition transducer arrays, a microbeamformer subassembly, a multiplexer assembly, a transducer controller assembly subassembly, selection means for indicating a selected one of the plurality of ultrasonic image data acquisition transducer arrays to the transducer controller assembly, and a communications assembly for communicating with a host system. The plurality of ultrasonic image data acquisition transducer arrays could be of different types (not being part of the claimed invention), have different operating characteristics, and/or have different modes of operation.

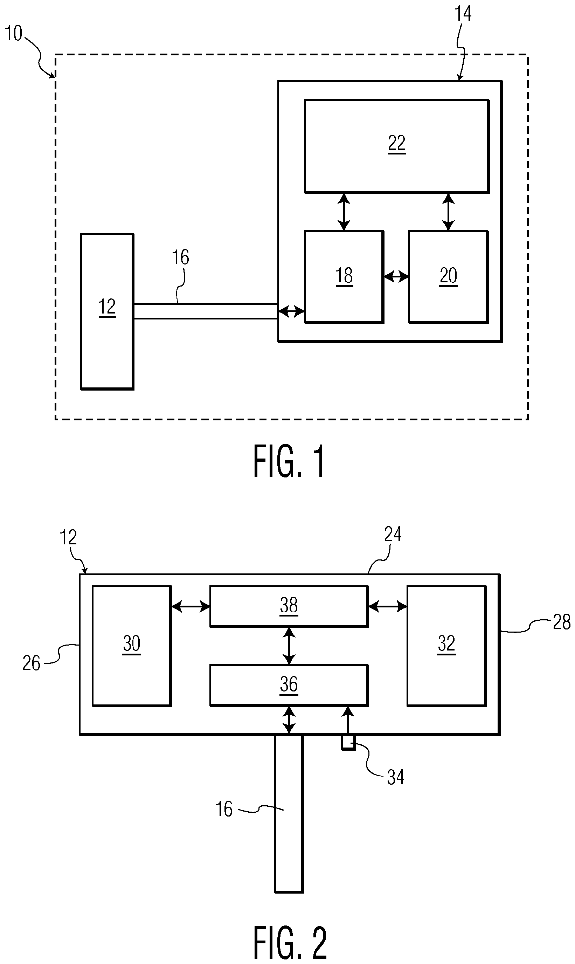

Referring now to FIG. 1, an exemplary ultrasonic examination system is generally indicated at 10. The ultrasonic examination system 10 includes an ultrasonic transducer assembly 12 that is in electrical communication with a host system 14 through a communications assembly 16. In some embodiments, the communications assembly 16 includes a multi-conductor cable and/or a wireless interface.

The host system 14 processes ultrasound image data provided by the transducer assembly 12 and generates corresponding images. The host system 14 -- which generally includes a central processing unit (CPU) and/or controller -- also includes for purposes of the present disclosure a signal processing assembly 18 that is in electrical communication with a user interface 20 and a display 22. The signal processing assembly 18 generates transmit waveforms (not shown) and control data (not shown) that are transmitted to the transducer assembly 12. The signal processing assembly 18 acquires ultrasound image data from the transducer assembly 12 through the communications assembly 16 and provides corresponding image signals to the display 22 for presentation to a sonographer (not shown). The sonographer actuates the user interface 20 to control operating characteristics of the transducer assembly 12 and/or to control display characteristics of the display 22. Operating characteristics of the transducer assembly 12 could include center frequencies, axial foci, scan plane orientation, and fundamental versus harmonic performance, for example.

Referring now to FIG. 2, a schematic depiction of the transducer assembly 12 of FIG. 1 is shown. The transducer assembly 12 includes a hard polymeric housing 24 having a first end 26 and a second end 28. A first ultrasonic image data acquisition transducer array 30 is disposed with respect to the first end 26 of the housing 24. A second ultrasonic image data acquisition transducer array 32 is disposed with respect to the second end 28 of the housing 24. The ultrasonic image data acquisition transducer arrays 30, 32 could be formed by ceramic piezoelectric transducer elements, a piezoelectric polymer, such as polyvinylidene difluoride (PVDF), or a semiconductor-based micromachined ultrasound transducer (MUT) such as a piezoelectric MUT (PMUT) or a capacitive MUT (CMUT) array of elements, for example.

The ultrasonic image data acquisition transducer arrays 30, 32 could be of the same type or of different types (not being part of the claimed invention). Types of ultrasonic image data acquisition transducer arrays include phased arrays, linear arrays, and curved arrays, each of which could be operated such that acoustic signals are emitted and received by the transducer elements to produce radiation patterns in desired locations and with desired focal characteristics. Phased arrays generally scan a sector (e.g., pie-shaped) region by steering the beams from a stationary aperture, whereas linear arrays generally scan a rectangular region by translating a sub-aperture across the face of the array. Curved arrays also generally use a translation form of scanning, but such translation typically moves across a curved array. For purposes of the present disclosure, it is expressly noted that varying transducer types and/or designs may be employed, e.g., two-dimensional and one-dimensional arrays. Scanning functionality may be achieved through mechanical and/or electronic means, as are known in the art.

It is noted that both of the transducer arrays 30, 32 are ultrasonic image data acquisition transducer arrays. Prior art transducer assemblies have included a single ultrasonic image data acquisition transducer array and one or more tracking arrays that track the position of the transducer assembly. For example, U.S. Patent No. 6,142,946 (Hossack et al.) discloses a transducer assembly that includes a single ultrasonic image data acquisition transducer array and two tracking arrays. There is no selection among the arrays as all of the arrays are operated concurrently and only one array is capable of acquiring ultrasound image data.

A switch 34 is disposed with respect to the housing 24. A sonographer (not shown) could actuate the switch 34 to select one of the ultrasonic image data acquisition transducer arrays 30, 32 for operation. The switch 34 is in electrical communication with a transducer controller assembly 36, which is in electrical communication with a multiplexer assembly 38. The multiplexer assembly 38 also is in electrical communication with multiplexed arrays of the ultrasonic image data acquisition transducer array 30, 32.

Actuation of the switch 34 causes the transducer controller assembly 36 to provide the multiplexer assembly 38 with transmit waveform signals and multiplexer control signals appropriate for the selected image data acquisition transducer array 30, 32. Multiplexed arrays of the selected image data acquisition transducer array 30, 32 provide transducer signals to the multiplexer assembly 38, which provides the transducer signals to the transducer controller assembly 36. For example, if the switch 34 is positioned to select the ultrasonic image data acquisition transducer array 30, the transducer controller assembly 36 provides transmit waveform signals and multiplexer control signals to the multiplexer assembly 38 appropriate for selection of multiplexed arrays of transducer elements of the first image data acquisition transducer array 30.

The multiplexer assembly 38 provides transducer signals received from the selected ultrasonic image data acquisition transducer array 30, 32 to the transducer controller assembly 36. The transducer controller assembly 36 transmits the transducer signals through the communications assembly 16 to the host system 14 (shown in FIG. 1), where the image data are processed and displayed.

The following example illustrates an exemplary configuration and use of the ultrasonic transducer assembly 12 shown in FIG. 2. The ultrasonic transducer assembly 12 includes two identical types of ultrasonic image data acquisition transducer arrays 30, 32. Initially, a sonographer (not shown) actuates the switch 34 to select the first ultrasonic image data acquisition transducer array 30 and then performs an ultrasonic imaging operation for a period of time until surface temperature limits dictate that the image data acquisition transducer array 30 be inactive for a period of time to allow it to cool down.

The probe control assembly 36 contains a temperature sensor (not shown) that activates an audio and/or visual alarm (not shown) when the temperature of the selected image data acquisition transducer array 30, 32 exceeds a predetermined threshold. Once the alarm has been activated, the sonographer positions the switch 34 to activate the second ultrasonic image data acquisition transducer array 32 and continues performing the ultrasonic imaging operation without waiting for the first image data acquisition transducer array 30 to cool down and without changing the transducer assembly 12.

In some embodiments, the switch 34 is replaced by circuitry that automatically determines which of the ultrasonic image data acquisition transducer arrays 30, 32 is being used. For example, the switch 34 could be replaced by circuitry that detects reflections from lens-air interfaces (not shown) in front of the ultrasonic image data acquisition transducer arrays 30, 32 to determine if either is being used. U.S. Patent Nos. 4,603,702 (Hwang et al.) and 5,654,509 (Miele et al.) disclose technologies suitable for automatically determining which of the ultrasonic image data acquisition transducer arrays 30, 32 is being used for imaging.

Alternatively, the transducer controller assembly 36 could cause the ultrasonic image data acquisition transducer arrays 30, 32 to periodically perform Doppler scanning, even if not in a Doppler mode, to determine if blood flow movement is scanning, even if not in a Doppler mode, to determine if blood flow movement is detected, thereby indicating which one, if any, of the image data acquisition transducer arrays 30, 32 is in use.

Moreover, in some embodiments, the switch 34 is disposed with respect to the host system 14. When the switch 34 is disposed with respect to the host system 14, the signal processing assembly 18 provides a transducer array type indicator to the transducer controller assembly 36 to ensure that the transducer controller assembly 36 communicates with the selected one of the ultrasonic image data acquisition transducer arrays 30, 32.

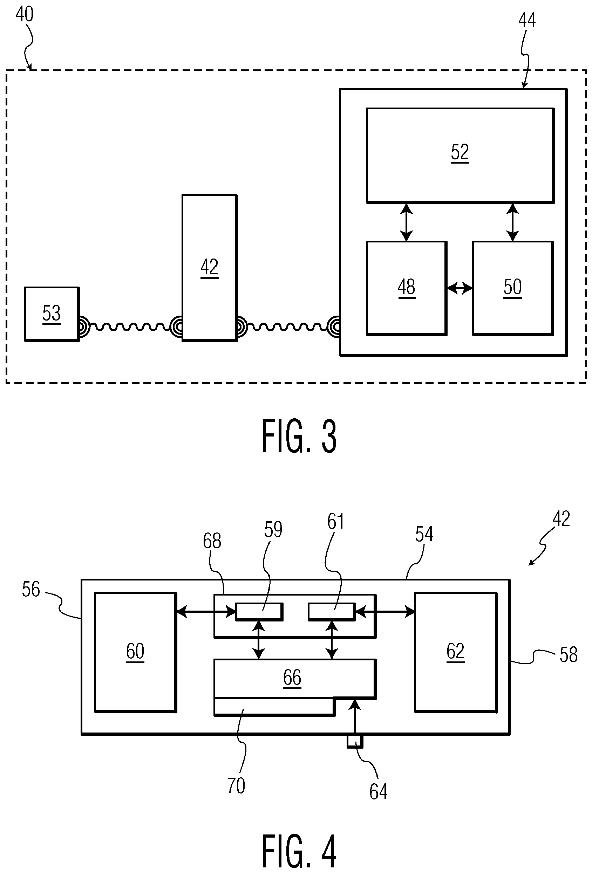

Referring now to FIG. 3, another exemplary ultrasonic examination system is generally indicated at 40. The ultrasonic examination system 40 includes an ultrasonic transducer assembly 42 that is in wireless communication with a host system 44. The ultrasonic examination system 40 also includes a remote control assembly 53 that is in wireless communication with the ultrasonic transducer assembly 42 and/or the host system 44.

The host system 44 includes a signal processing assembly 48 that is in wireless communication with the transducer assembly 42. The signal processing assembly 48 is also in electrical communication with a user interface 50 and a display 52. The signal processing assembly 48 wirelessly provides transmit waveform data as well as control data to the transducer assembly 42. The ultrasonic transducer assembly 42 wirelessly provides ultrasound image data (not shown) to the signal processing assembly 48 of host system 44 for processing and display.

It is noted that, in providing microbeamformed arrays, typically such arrays are provided as either (i) actual transmit waveforms (i.e., in direct analog form), or (ii) a parameterized version of the waveform (i.e., digital values reflecting relevant parameters, e.g., center frequency, number of cycles, delay, envelope shape and the like). For wireless communication modalities, the latter waveform may be utilized to advantage, at least in part because (i) the wireless channel is digital, and (ii) bandwidth limitations are better accommodated using parameterized waveforms. Of note, bandwidth limitations generally do not arise in cable-based communication modalities, thereby accommodating transmission of actual transmit waveforms. Thus, the present
disclosure advantageously provides transmit waveform data (in either direct or parameterized form), as well as control data.

The signal processing assembly 48 processes the acquired ultrasound image data and provides corresponding image signals to the display 52 for presentation to a sonographer (not shown). The sonographer could actuate the user interface 50 to control operating characteristics of the transducer assembly 42 and/or to control display characteristics of the display 52. Further, the sonographer could actuate the remote control assembly 53 to control the operating characteristics and/or mode of operation of the transducer assembly 42.

Referring now to FIG. 4, a schematic depiction of the transducer assembly 42 of FIG. 3 is shown. The ultrasonic transducer assembly 42 includes a hard polymeric housing 54 having a first end 56 and a second end 58. A first ultrasonic image data acquisition transducer array 60 is disposed with respect to the first end 56 of the housing 54. A second ultrasonic image data acquisition transducer array 62 is disposed with respect to the second end 58 of the housing 54. The ultrasonic image data acquisition transducer arrays 60, 62 could be formed by ceramic piezoelectric transducer elements, a piezoelectric polymer, or a semiconductor-based MUT such as a PMUT or a CMUT array of elements, for example.

The ultrasonic image data acquisition transducer arrays 60, 62 could be of the same type or of different types (not being part of the claimed invention). Types of ultrasonic image data acquisition transducer arrays include planar arrays, linear arrays, and curved arrays, each of which could be operated as a phased array wherein relative phases of acoustic signals emitted and received by the transducer elements are varied to produce radiation patterns in desired locations and with desired focal characteristics.

A switch 64 is disposed with respect to the housing 54. A sonographer (not shown) actuates the switch 64 to select one of the ultrasonic image data acquisition transducer arrays 60, 62 for operation. The switch 64 is in electrical communication with a transducer controller assembly 66 that is in electrical communication with a microbeamformer assembly 68. The microbeamformer assembly 68
includes a first microbeamformer 59 and a second microbeamformer 61. The first microbeamformer 59 is in electrical communication with the first ultrasonic image data acquisition transducer
array 60 and the second microbeamformer 61 is in electrical communication with the second ultrasonic image data acquisition transducer array 62.

The transducer controller assembly 66 includes a wireless communications assembly 70 through which transmit waveform descriptions and control data are received from the host system 44 (shown in FIG. 3) and/or the remote control assembly 53 (shown in FIG. 3). The wireless communications assembly 70 is also used to transmit ultrasound image data to the signal processing assembly 48 (shown in FIG. 3) of the host system 44. The disclosed wireless communication assembly 70 generally includes one or more antennae/transceivers to facilitate transmission and receipt of wireless communications thereby. A plurality of antennae may be particularly advantageous in the disclosed wireless communication assembly 70, e.g., to counteract potential multipathing in the wireless communication process and/or to avoid inadvertent shielding or communication difficulties associated with a communication obstacle, e.g., based on the positioning of an operator's hand.

The microbeamformer assembly 68 generates beamformed signals by applying delays and combining per-element transducer signals into a small number of beamformed signals. The control data could specify delay values to be used by the microbeamformer assembly 68, for example. For example, the first ultrasonic image data acquisition transducer array 60 could be comprised of 128 individual transducer elements (not shown) and the first microbeamformer assembly 59 receives transducer signals from the individual transducer elements, applies specified delays, and combines the received transducer signals to form eight partially beamformed signals.

The microbeamformer assembly 68 could also be implemented to produce fully beamformed signals from all transducer elements of an active aperture as described in the U.S. Patent No. 6,142,946 (Hwang et al.). Microbeamformer technology suitable for use in microbeamformer assembly 68 is described in U.S. Patent Nos. 5,229,933 (Larson III), 5,997,479 (Savord et al.), and 6,375,617 (Fraser).

In some embodiments, the transducer controller assembly 66 performs secondary beamforming operations, signal conditioning, band-pass filtering, detection, analog to digital operations, post-filtering operations, and/or compression on received beamformed transducer signals and provides the resulting image data to the host system 44.

The transducer controller assembly 66 receives transmit waveform descriptions and control signals from the host system 44 and provides corresponding transmit waveform description signals and beamformer control signals to one of the microbeamformers 59, 61 that is connected to the selected one of the ultrasonic image data acquisition transducer arrays 60, 62. For example, the host system 44 could provide transmit waveform descriptions and control data through the wireless communications assembly 70 to the transducer controller assembly 66, which provides corresponding transmit waveform description signals and beamformer control signals to the microbeamformer assembly 68 to focus beams at a desired depth or transmit and receive signals of a desired mode to and from a desired region of an image.

The ultrasonic transducer assembly 42 could include any combination of transducer array types (not claimed), modes of operation, and/or operating characteristics. Exemplary modes of operation include fundamental imaging, harmonic imaging, B-mode imaging, pulsed Doppler, CW Doppler, and color Doppler imaging.

The following example illustrates an exemplary configuration and use of the ultrasonic transducer assembly 42 shown in FIG. 4. The ultrasonic transducer assembly 42 could include two different types of ultrasonic image data acquisition transducer arrays 60, 62 (not claimed). The first ultrasonic image data acquisition transducer array 60 could include a curved array of transducer elements (not shown). The second ultrasonic image data acquisition transducer array 62 could include a linear array of transducer elements operated as a phased array.

Initially, the sonographer (not shown) actuates the switch 64 to activate the first ultrasonic image data acquisition transducer array 60, which causes the transducer controller assembly 66 to provide transmit waveform description signals and control signals received by the wireless communications assembly 70 to the first microbeamformer 59. Actuation of the switch 64 also causes the transducer controller assembly 66 to receive from the first microbeamformer 59 beamformed transducer signals, which are processed by controller assembly 66 and provided to the wireless communications assembly 70 for wireless transmission to the host system 44. Further, actuation of the switch 64 causes the transducer controller assembly 66 to transmit a transducer array type indicator (not shown) to the host system 44 (shown in FIG. 3) and/or the remote control assembly 53 (shown in FIG. 3) to ensure that appropriate transmit waveform descriptions and control data are supplied to the transducer assembly 42.

After performing a first portion of an ultrasonic examination, the sonographer actuates the switch 64 to activate the second ultrasonic image data acquisition transducer array 62, which causes the transducer controller assembly 66 to transmit another transducer array type indicator to the host system 44 (shown in FIG. 3) and/or the remote control assembly 53 (shown in FIG. 3) to ensure that appropriate transmit waveform descriptions and control data will be provided to the transducer assembly 42. The microbeamformer assembly 68 now provides transmit waveform description signals and control signals received by the wireless communications assembly 70 to the second microbeamformer 61. Actuation of the switch 64 also causes the transducer controller assembly 66 to receive from the second microbeamformer 61 beamformed transducer signals, which are processed by the controller assembly 66 and provided to the wireless communications assembly 70 for wireless transmission to the host system 44. The sonographer then performs a second portion of the ultrasonic examination using the second ultrasonic image data acquisition transducer array 62.

Accordingly, the ultrasonic transducer assembly 42 advantageously allows the sonographer to perform an ultrasonic examination using two different types of ultrasonic image data acquisition transducer arrays 60, 62 without having to change the transducer assembly 42. Further, the sonographer is required to bring fewer ultrasonic transducer assemblies 42 to the examination location. Still further, there is no need to store a used transducer assembly 42 during the examination. In addition, the overall system cost is reduced as many components are shared by more than one ultrasonic image data acquisition transducer array, for example, the housing 54, the transducer controller assembly 66, the wireless communications assembly 70, and power supply (not shown) could be shared by the ultrasonic image data acquisition transducer arrays 60, 62

In some embodiments, the switch 64 is disposed with respect to the host system 44 (shown in FIG. 3) or the remote control assembly 53 (shown in FIG. 3). A transducer type indicator is wirelessly communicated through the wireless communications assembly 70 to the transducer controller assembly 44, which causes the transducer controller assembly 44 to provide transmit waveform description signals and control signals to one of the microbeamformers 59, 61 connected to the selected one of the ultrasonic image data acquisition transducer arrays 60, 62. In other embodiments the switch 64 is replaced by circuitry that automatically determines which of the ultrasonic image data acquisition transducer arrays 60, 62 , if any, is being used for imaging, as discussed above.

## Claims

1. An ultrasonic transducer assembly (12), comprising:
a housing (24) having a first end (26) and a second end (28);
a first and a second discrete ultrasonic image data acquisition transducer array (30, 32) disposed with respect to said housing (24);
a transducer controller assembly (36) disposed with respect to said housing (24) and in electrical communication with each of said ultrasonic image data acquisition transducer arrays (30, 32);
a communications assembly (16) disposed with respect to said housing (24) and in electrical communication with said transducer controller assembly (36); and
selection means (34) for indicating a selected one of said ultrasonic image data acquisition transducer arrays (30, 32) to said transducer controller assembly (36), wherein said transducer controller assembly (36) provides transmit waveform data received by said communications assembly (16) to said selected one of said ultrasonic image data acquisition transducer arrays (30, 32), said transducer controller assembly (36) providing transducer signals from said selected one of said ultrasonic image data acquisition transducer arrays (30, 32) to said communications assembly (16), wherein the transducer controller assembly (36) further comprises a temperature sensor arranged to activate an audio and/or visual alarm, when the temperature of the selected ultrasonic image data acquisition transducer array exceeds a predetermined threshold; and wherein the first discrete ultrasonic image data acquisition transducer array (30) is disposed at the first end (26) of the housing (24) and the second discrete ultrasonic image data acquisition transducer array (32), which is of identical type as the first discrete ultrasonic image data acquisition transducer array (30), is disposed at the second end (28) of the housing (24).

2. The ultrasonic transducer assembly (12) according to claim 1, wherein said communications assembly (16) includes a cable.

3. The ultrasonic transducer assembly (12) according to claim 1, wherein said communications assembly (16) includes a wireless interface.

4. The ultrasonic transducer assembly (12) according to claim 1, wherein said selection means (34) automatically selects one of said ultrasonic image data acquisition transducer arrays (30, 32).

5. An ultrasonic transducer assembly (12), comprising:
a housing (24) having a first end (26) and a second end (28);
a first and a second discrete ultrasonic image data acquisition transducer array (30, 32) disposed with respect to said housing (24);
a multiplexer assembly (38) disposed with respect to said housing (24) and in electrical communication with each of said ultrasonic image data acquisition transducer arrays (30, 32);
a transducer controller assembly (36) disposed with respect to said housing (24) and in electrical communication with said multiplexer assembly (38);
a communications assembly (16) within said housing (24) and in electrical communication with said transducer controller assembly (36); and
selection means (34) for indicating a selected one of said ultrasonic image data acquisition transducer arrays (30, 32) to said transducer controller assembly (36), wherein said transducer controller assembly (36) provides transmit waveform data and control data received by said communications assembly (16) to said multiplexer assembly (38), said multiplexer assembly (38) providing said transmit waveform data to said selected one of said ultrasonic image data acquisition transducer arrays (30, 32), said multiplexer assembly (38) providing transducer signals received from said selected one of said ultrasonic image data acquisition transducer arrays (30, 32) to said transducer controller assembly (36), said transducer controller assembly (36) providing said transducer signals to said communications assembly (16), wherein the transducer controller assembly (36) further comprises a temperature sensor arranged to activate an audio and/or visual alarm, when the temperature of the selected ultrasonic image data acquisition transducer array exceeds a predetermined threshold; and
wherein the first discrete ultrasonic image data acquisition transducer array (30) is disposed at the first end (26) of the housing (24) and the second discrete ultrasonic image data acquisition transducer array (32), which is of identical type as the first discrete ultrasonic image data acquisition transducer array (30), is disposed at the second end (28) of the housing (24).

6. The ultrasonic transducer assembly (12) according to claim 5, wherein said selection means (34) automatically selects one of said ultrasonic image data acquisition transducer arrays (30, 32).

7. The ultrasonic transducer assembly (12) according to claims 1 or 5, wherein the communications assembly includes a multi-conductor cable.

8. The ultrasonic transducer assembly (12) according to claim 5, wherein the communications assembly includes a wireless interface.

## Patentansprüche

1. Ultraschallwandler-Einheit (12), umfassend:
ein Gehäuse (24) mit einem ersten Ende (26) und einem zweiten Ende (28); eine erste und eine zweite diskrete Ultraschallbild-Datenerfassungs-Wandleranordnung (30, 32), die in Bezug auf das Gehäuse (24) angeordnet sind;
eine Wandlersteuereinheit (36), die in Bezug auf das Gehäuse (24) angeordnet ist und in elektrischer Verbindung mit jeder der Ultraschall-Bilddatenerfassungs-Wandleranordnungen (30, 32) steht;
eine Kommunikationseinheit (16), die in Bezug auf das Gehäuse (24) angeordnet ist und in elektrischer Verbindung mit der Wandlersteuereinheit (36) steht; und
Auswahlmittel (34) zum Anzeigen einer ausgewählten der Ultraschall-Bilddatenerfassungs-Wandleranordnungen (30, 32) an die Wandlersteuereinheit (36), wobei die Wandlersteuereinheit (36) eine Sendewellenformdaten, die von der Kommunikationseinheit (16) empfangen werden, an die ausgewählte der Ultraschall-Bilddatenerfassungs-Wandleranordnungen (30, 32) bereitstellt, wobei die Wandlersteuereinheit (36) Wandlersignale von der ausgewählten der Ultraschall-Bilddatenerfassungs-Wandleranordnungen (30, 32) an die Kommunikationseinheit (16) bereitstellt, wobei die Wandlersteuereinheit (36) weiter einen Temperatursensor umfasst, der zum Aktivieren eines Audio- und/oder visuellen Alarms angeordnet ist, wenn die Temperatur der ausgewählten Ultraschall-Bilddatenerfassungs-Wandlereinheit einen vorbestimmten Schwellenwert überschreitet; und wobei eine erste diskrete Ultraschall-Bilddatenerfassungs-Wandleranordnung (30) an dem ersten Ende (26) des Gehäuses (24) angeordnet ist und die zweite diskrete Ultraschall-Bilddatenerfassungs-Wandleranordnung (32), die vom identischen Typ wie die erste diskrete Ultraschall-Bilddatenerfassungs-Wandleranordnung (30) ist, an dem zweiten Ende (28) des Gehäuses (24) angeordnet ist.

2. Ultraschallwandler-Einheit (12) nach Anspruch 1, wobei die Kommunikationseinheit (16) ein Kabel aufweist.

3. Ultraschallwandler-Einheit (12) nach Anspruch 1, wobei die Kommunikationseinheit (16) eine drahtlose Schnittstelle aufweist.

4. Ultraschallwandler-Einheit (12) nach Anspruch 1, wobei das Auswahlmittel (34) automatisch eine der Ultraschall-Bilddatenerfassungs-Wandleranordnungen (30, 32) auswählt.

5. Ultraschallwandler-Einheit (12), umfassend:
ein Gehäuse (24) mit einem ersten Ende (26) und einem zweiten Ende (28); eine erste und eine zweite diskrete Ultraschallbild-Datenerfassungs-Wandleranordnung (30, 32), die in Bezug auf das Gehäuse (24) angeordnet sind;
eine Multiplexer-Einheit (38), die in Bezug auf das Gehäuse (24) angeordnet ist und in elektrischer Verbindung mit jeder der Ultraschall-Bilddatenerfassungs-Wandleranordnungen (30, 32) steht;
eine Wandlersteuereinheit (36), die in Bezug auf das Gehäuse (24) angeordnet ist und in elektrischer Verbindung mit der Multiplexer-Einheit (38) steht;
eine Kommunikationseinheit (16) innerhalb des Gehäuses (24) angeordnet ist und in elektrischer Verbindung mit der Wandlersteuereinheit (36) steht; und
Auswahlmittel (34) zum Anzeigen einer ausgewählten der Ultraschall-Bilddatenerfassungs-Wandleranordnungen (30, 32) an die Wandlersteuereinheit (36), wobei die Wandlersteuereinheit (36) Sendewellenformdaten und Steuerdaten, die von der Kommunikationseinheit (16) empfangen werden, an die Multiplexer-Einheit (38) bereitstellt, die Multiplexer-Einheit (38) die Sendewellenformdaten an die ausgewählte der Ultraschall-Bilddatenerfassungs-Wandleranordnungen (30, 32) bereitstellt, die Multiplexer-Einheit (38) Wandlersignale, die von der ausgewählten der Ultraschall-Bilddatenerfassungs-Wandleranordnungen (30, 32) empfangen werden, an die Wandlersteuereinheit (36) bereitstellt, die Wandlersteuereinheit (36) die Wandlersignale der Kommunikationseinheit (16) bereitstellt, wobei die Wandlersteuereinheit (36) weiter einen Temperatursensor umfasst, der zum Aktivieren eines Audio- und/oder visuellen Alarms angeordnet ist, wenn die Temperatur der ausgewählten Ultraschall-Bilddatenerfassungs-Wandlereinheit einen vorbestimmten Schwellenwert überschreitet; und wobei die erste diskrete Ultraschall-Bilddatenerfassungs-Wandleranordnung (30) an dem ersten Ende (26) des Gehäuses (24) angeordnet ist und die zweite diskrete Ultraschall-Bilddatenerfassungs-Wandleranordnung (32), die vom identischen Typ wie die erste diskrete Ultraschall-Bilddatenerfassungs-Wandleranordnung (30) ist, an dem zweiten Ende (28) des Gehäuses (24) angeordnet ist.

6. Ultraschallwandler-Einheit (12) nach Anspruch 5, wobei das Auswahlmittel (34) automatisch eine der Ultraschall-Bilddatenerfassungs-Wandleranordnungen (30, 32) auswählt.

7. Ultraschallwandler-Einheit (12) nach einem der Ansprüche 1 oder 5, wobei die Kommunikationseinheit ein Mehrfachleiterkabel aufweist.

8. Ultraschallwandler-Einheit (12) nach Anspruch 5, wobei die Kommunikationseinheit eine drahtlose Schnittstelle aufweist.

## Revendications

1. Ensemble transducteur ultrasonore (12) comprenant :
un boîtier (24) ayant une première extrémité (26) et une seconde extrémité (28) ;
un premier et un second réseau de transducteurs d'acquisition de données d'image ultrasonores discrets (30, 32) disposés par rapport audit boîtier (24) ;
un ensemble contrôleur de transducteur (36) disposé par rapport audit boîtier (24) et en communication électrique avec chacun desdits réseaux de transducteurs d'acquisition de données d'image ultrasonores (30, 32) ;
un ensemble de communications (16) disposé par rapport audit boîtier (24) et en communication électrique avec ledit ensemble contrôleur de transducteur (36) ; et
des moyens de sélection (34) pour indiquer l'un desdits réseaux sélectionné desdits réseaux de transducteurs d'acquisition de données d'image ultrasonores (30, 32) audit ensemble contrôleur de transducteur (36), dans lequel ledit ensemble contrôleur de transducteur (36) fournit des données de forme d'onde d'émission reçues par ledit ensemble de communications (16) audit un réseau sélectionné desdits réseaux de transducteurs d'acquisition de données d'image ultrasonores (30, 32), ledit ensemble contrôleur de transducteur (36) fournissant des signaux de transducteur issus dudit un réseau sélectionné desdits réseaux de transducteurs d'acquisition de données d'image ultrasonores (30, 32) audit ensemble de communications (16),
dans lequel l'ensemble contrôleur de transducteur (36) comprend en outre un capteur de température conçu pour activer une alarme sonore et/ou visuelle, lorsque la température du réseau de transducteurs d'acquisition de données d'image ultrasonores sélectionné dépasse un seuil prédéterminé ; et dans lequel le premier réseau de transducteurs d'acquisition de données d'image ultrasonores discrets (30) est disposé à la première extrémité (26) du boîtier (24) et le second réseau de transducteurs d'acquisition de données d'image ultrasonores discrets (32), qui est du même type que le réseau de transducteurs d'acquisition de données d'image ultrasonores discrets (30), est disposé à la seconde extrémité (28) du boîtier (24).

2. Ensemble transducteur ultrasonore (12) selon la revendication 1, dans lequel ledit ensemble de communications (16) inclut un câble.

3. Ensemble transducteur ultrasonore (12) selon la revendication 1, dans lequel ledit ensemble de communications (16) inclut une interface sans fil.

4. Ensemble transducteur ultrasonore (12) selon la revendication 1, dans lequel ledit moyen de sélection (34) sélectionne automatiquement l'un desdits réseaux de transducteurs d'acquisition de données d'image ultrasonores (30, 32).

5. Ensemble transducteur ultrasonore (12), comprenant :
un boîtier (24) ayant une première extrémité (26) et une seconde extrémité (28) ;
un premier et un second réseau de transducteurs d'acquisition de données d'image ultrasonores discrets (30, 32) disposés par rapport audit boîtier (24) ;
un ensemble multiplexeur (38) disposé par rapport audit boîtier (24) et en communication électrique avec chacun desdits réseaux de transducteurs d'acquisition de données d'image ultrasonores (30, 32) ;
un ensemble contrôleur de transducteur (36) disposé par rapport audit boîtier (24) et en communication électrique avec ledit ensemble multiplexeur (38) ;
un ensemble de communications (16) dans ledit boîtier (24) et en communication électrique avec ledit ensemble contrôleur de transducteur (36) ; et
des moyens de sélection (34) pour indiquer un réseau sélectionné desdits réseaux transducteurs d'acquisition de données d'image ultrasonores (30, 32) audit ensemble contrôleur de transducteur (36), dans lequel ledit ensemble contrôleur de transducteur (36) fournit des données de forme d'onde d'émission et des données de contrôle reçues par ledit ensemble de communications (16) audit ensemble multiplexeur (38), ledit ensemble multiplexeur (38) fournissant lesdites données de forme d'onde d'émission audit réseau sélectionné desdits réseaux de transducteurs d'acquisition de données d'image ultrasonores (30, 32), ledit ensemble multiplexeur (38) fournissant des signaux de transducteur reçus dudit réseau sélectionné desdits réseaux de transducteurs d'acquisition de données d'image ultrasonores (30, 32) audit ensemble contrôleur de transducteur (36), ledit ensemble contrôleur de transducteur (36) fournissant lesdits signaux de transducteur audit ensemble de communications (16), dans lequel l'ensemble contrôleur de transducteur (36) comprend en outre un capteur de température conçu pour activer une alarme sonore et/ou visuelle, lorsque la température du réseau de transducteurs d'acquisition de données d'image ultrasonores sélectionné dépasse un seuil prédéterminé ; et
dans lequel le premier réseau de transducteurs d'acquisition de données d'image ultrasonores discrets (30) est disposé à la première extrémité (26) du boîtier (24) et le second réseau de transducteurs d'acquisition de données d'image ultrasonores discrets (32), qui est du même type que le premier réseau de transducteurs d'acquisition de données d'image ultrasonores discrets (30), est disposé à la seconde extrémité (28) du boîtier (24).

6. Ensemble transducteur ultrasonore (12) selon la revendication 5, dans lequel ledit moyen de sélection (34) sélectionne automatiquement l'un desdits réseaux de transducteurs d'acquisition de données d'image ultrasonores (30, 32).

7. Ensemble transducteur ultrasonore (12) selon la revendication 1 ou 5, dans lequel l'ensemble de communications inclut un câble multiconducteur.

8. Ensemble transducteur ultrasonore (12) selon la revendication 5, dans lequel l'ensemble de communications inclut une interface sans fil.
